# EUROPEAN PATENT APPLICATION

(11) **EP 1 745 777 A1**
(43) Date of publication of application: **24.01.2007**
(21) Application number: 06117473.6
(22) Date of filing: 19.07.2006
(51) Int. Cl.: A61K 31/132, A61K 31/166, A61P 25/28, A61P 31/18, A61P 33/06

(54) **Amido-aminoalcohols as therapeutic compounds**

(30) Priority: 20.07.2005 CH 12112005
(71) Applicant: Speedel Experimenta AG, 4123 Allschwil (CH)
(72) Inventor: Herold, Peter, 4055, Basel (CH); Stutz, Stefan, 4053, Basel (CH); Tschinke, Vincenzo, 4102, Binningen (CH); Stojanovic, Aleksandar, 4055, Basel (CH); Marti, Christiane, 5400, Baden (CH); Quirmbach, Michael, 4054, Basel (CH); Schumacher, Christoph, 4126, Bettingen (CH)
(74) Representative: Dannappel, Hans-Jochen

(57) **Abstract**

Use of compounds of the general formula (I) 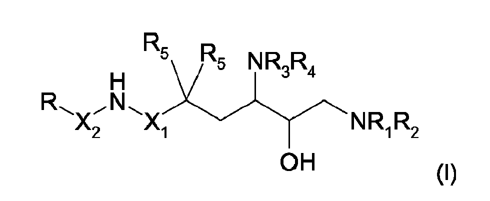 in which R, R₁, R₂, R₃, R₄, R₅, X₁ and X₂ have the definitions illustrated in detail in the description, as beta-secretase, cathepsin D, plasmepsin II and/or HIV protease inhibitors.

## Description

The present invention relates to the use of aminoalcohols as beta-secretase-, cathepsin D-, plasmepsin II- and/or HIV-protease-inhibitors.

With regard to beta-secretase-, cathepsin D-, plasmepsin II- and/or HIV-protease-inhibition, there is still a need for highly potent active ingredients. In this context, the improvement of the pharmacokinetic properties is at the forefront. These properties directed towards better bioavailability are, for example, absorption, metabolic stability, solubility or lipophilicity.

### Alzheimer Disease aspartyl protease: Beta-Secretase

Alzheimer's disease (AD) is a progressive degenerative disease of the brain. The symptoms of AD include progressive memory loss, language difficulty and ultimately loss of basic neural function and death. The biomarkers in the central nervous system for AD include amyloid plaques, intracellular neurofibrillary tangles and activated microglia. The appearance of these three markers is likely to contribute to the neuronal cell death and memory loss observed in AD.

Beta-amyloid is a defining feature of AD and now believed to be a causative precursor in the development of the disease. Amyloidogenic plaques and vascular amyloid angiopathy also characterize the brains of individuals with Trisomy 21 (Down's Syndrome), Hereditary Cerebral Hemorrhage with Amloidosis of the Dutch-Type (HCHWA-D) and other neurodegenerative disorders.

Beta-amyloid plaques are predominantly composed of amyloid beta peptide (A-beta, also sometimes designated betaA4). The A-beta peptide is derived by proteolysis of the beta amyloid precursor protein (APP). Beta-APP is processed by three distinct ordered enzymatic activities. The bulk of beta-APP is processed via alpha-secretase in a non-amyloidogenic pathway. A small fraction of beta-APP is cleaved by beta-secretase activity to generate the membrane-bound C-terminal fragment C99. Gamma-secretase cleaves C99 to generate the amyloidogenic A-beta peptide of 39-42 amino acids. The aspartyl protease activity of beta-secretase has been disclosed using varied nomenclature, including BACE (beta-site APP cleaving enzyme), Asp and memapsin.

The significance of beta-secretase cleavage of beta-APP as a critical step in the generation of AD is underscored by the observation that human mutations at the beta-secretase cleavage subsites (Swedish mutations) of beta-APP lead to increased A-beta production and early onset familial AD. Furthermore, BACE1- knockout mice fail to produce A-beta peptide and present a normal phenotype. When crossed with transgenic mice that overexpress APP, the progeny show reduced amounts of A-beta in brain extracts as compared with control animals. This evidence supports the proposal that inhibition of beta-secretase activity and reduction of A-beta peptide deposits in the brain provides a therapeutic strategy for the treatment of AD and other beta amyloid disorders as described by Verdile et al. (2004) in Pharmacol. Res 50, 397-409.

Compounds that are effective inhibitors of beta-secretase may inhibit beta-secretase-mediated cleavage of APP and the production of A-beta peptide. The pharmacological inhibition of A-beta peptide generation may reduce amyloid beta deposits, respectively the formation of plaques. Beta-secretase inhibiting compounds as discussed by Thompson et al. (2005) in Curr. Pharm. Des. 11, 3383-3404 are therefore useful to treat or to prevent diseases that are characterized by amyloid beta deposits or plaques such as AD.

The present invention also relates to methods of treating subjects who have, or in preventing subjects from developing a disease or condition selected from the group consisting of AD, for helping prevent or delay the onset of AD, for helping to slow the proression of AD, for treating subjects with mild cognitive impairment (MCI) and preventing or delaying the onset of AD in those who could progress form MCI to AD, for treating Down's syndrome, for treating humans who have HCHWAD, for treating cerebral amyloid angiopathy, and for treating degenerative dementias

### Alzheimner's Disease aspartyl protease: Cathepsin D

Human cathepsin D is an intracellular aspartic peptidase found mainly in lysosomes. It has a number of housekeeping functions, including the degradation of cellular and phagocytosed proteins. The enzymes may be involved in a variety of disease states, including cancer and Alzheimer's disease (AD). Clinical studies have shown that cathepsin D is overexpressed in breast cancer cells and this seems to be associated with an increased risk for metastasis due to enhanced cell growth. Cathepisn D is also thought to be involved in formation of the beta-amyloid peptide in AD. Recently, several genetic association studies linked cathepsin D with amyloid pathology and Alzheimer's disease as described for example by Davidson et al., (2006) in J. Neurol. Neurosurg. Psychiatry 77, 515-517. The availability of selective and potent inhibitors will help to further define the role of cathepsin D in disease and possibly lead to therapeutic agents.

### Malaria Aspartyl Protease: Plasmepsin I and II

Malaria is considered as one of the most serious infectious diseases in the world, affecting approximately 500 million people. The disease is spread by the anopheles mosquito that is mostly found in tropical regions. The species plasmodium falciparum is responsible for more than 95% of malaria-related morbidity and mortality. Increasingly, plasmodium falciparum is becoming resistant to existing therapies such as chloroquine, mefloquine and sulfadoxime/pyrimethamine. Thus there is an urgent need for new treatments.

In the erythrocytic stage of the parasite's life cycle the parasite invades the red blood cells of its host consuming up to 80% of the hemoglobin as a source of nutrients for growth and development. Hemoglobin degradation takes place in an acidic vacuole of the parasite and many of the current antimalarial drugs appear to disrupt important vacuolar functions. The food vacuole contains aspartic, cysteine and metallo-proteases, which are all considered to play a role in the process of hemoglobin degradation. At least 10 genes encoding aspartic proteases have been identified in the plasmodium genome. Four of the aspartic proteases have been localized in the acidic food vacuole of the parasite, namely plasmepsin I, II, IV and HAP, a histo-aspartic protease. Inhibitors of plasmepsin I and II have shown efficacy in cell and animal models of malaria, indicating that these enzymes may represent targets for drug discovery as described for example by Coombs et al. (2001) Trends Parasitol 17, 532-537. Indeed, a non-selective inhibitor of aspartic proteases, pepstatin, inhibits the growth of plasmodium falciparum in vitro. Similar results have been obtained with analogs of pepstatin or with immunodeficiency virus protease inhibitors indicating that inhibition of aspartic proteases interferes with the life cycle of plasmodium falciparum as noted for example by Andrews et al. (2006) in Antimicrob. Agents Chemother 50, 639-648.

The present invention relates to the identification of low molecular weight, non-peptidic inhibitors of the plasmodium falciparum protease plasmepsin II or other related aspartic proteases to treat and/or to prevent malaria.

### HIV aspartyl protease: HIV-1 peptidase

First reported in 1981 in a small number of patients, Acquired immunodeficiency syndrome (AIDS) has now become a major epidemic with more than 38 million people infected worldwide, including approximately 1 million in the United States, 580,000 in Western Europe and more than 25 million in Sub-Saharan Africa (http://www.unaids.org). Since AIDS was first clinically identified, scientific and therapeutic progress has been extraordinary. However, AIDS remains out of control, especially in developing countries.

The prognosis of AIDS patients who have full access to current therapies has completely changed since the first cases of AIDS were reported. Today, the median survival for HIV-positive patients receiving treatment exceeds 8 years. The life expectancy for AIDS patients was less than 1 year before AZT was introduced in 1987. This dramatic change is due to the development of effective therapies, to early detection of HIV-positive individuals, and to a sustained effort to analyze and understand viral-resistance mechanisms, which can be overcome by rational drug development and combination therapy.

FDA-approved therapies target three steps of the HIV life cycle: reverse transcription, proteolytic maturation and fusion. Triple therapy, commonly referred to as HIGHLY ACTIVE ANTIRETROVIRAL THERAPY (HAART), is now the standard for treatment. It consists of a protease inhibitor or a non-nucleoside reverse transcriptase inhibitor in combination with two nucleoside reverse transcriptase inhibitors.

Translation of human immunodeficiency virus type-1 (HIV-1) genomic RNA results in the production of two polyprotein precursors, Gag and Gag-Pol. The 55-kDa Gag precursor contains the structural proteins and the 160-kDa Gag-Pol polyprotein contains the functional viral enzymes protease, reverse transcriptase, and integrase. Gag and Gag-Pol polyproteins are transported to the plasma membrane where assembly of type-C retroviruses and lentiviruses typically occurs. During particle assembly, the viral protease cleaves the Gag and Gag-Pol precursors into the structural and functional proteins required for viral replication. The protease activity within the cytoplasma of infected cells allows for the formation of virions which can be released from the cell in the last stages of budding.

The mature HIV-1 protease is an obligatory dimer of identical 11-kDa subunits, each contributing one of the two catalytic aspartic residues. In contrast, the cell-derived members of the aspartic protease family are monomeric enzymes with two Asp-Thr-Gly-containing domains. The unique dimeric structure of the retroviral protease is mainly stabilized by an antiparallel beta-sheet formed by the interdigitation of the amino- and carboxyl-terminal beta-strands of each monomer.

The activation of HIV-1 protease i.e. the dimerization and autocatalytic release from Gag-Pol, is a critical step in the viral life cycle. Inhibition of protease activation causes a severe defect in Gag polyprotein processing and a complete loss of viral infectivity.

As such, the viral protease has become a target for HIV therapeutics, resulting in many HIV protease inhibitors reaching clinical trials as reviewed by Rana et al. (1999) in Pharmacotherapy 19, 35-59 and Morse et al., (2006) in Lancet Infect. Dis. 6, 215-225. Most of these drugs are substrate-based inhibitors, whose design has been facilitated by an abundance of crystal structure data for both the native enzyme and enzyme-inhibitor complexes. Additionally, there are now extensive biochemical data detailing both the catalytic mechanism and the molecular basis for substrate selection.

Firstly, the present invention relates to the use as beta-secretase-, cathepsin D-, plasmepsin II- and/or HIV-protease-inhibitors of compounds of the general formula 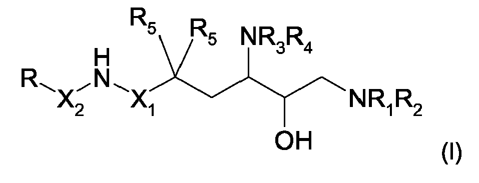 where
R₁ is a) hydrogen, hydroxyl or amino; or
is b) C₁-C₈-alkyl, C₃-C₈-cycloalkyl, C₁-C₈-alkanoyl, C₁-C₈-alkoxycarbonyl, aryl-C₀-C₄-alkyl or heterocyclyl-C₀-C₄-alkyl, which radicals may be substituted by 1-4 C₁-C₈-alkyl, halogen, cyano, oxide, oxo, trifluoromethyl, C₁-C₈-alkoxy, C₁-C₈-alkoxycarbonyl, aryl or heterocyclyl;
R₂ is a) C₁-C₈-alkyl, C₃-C₈-cycloalkyl, C₁-C₈-alkylsulphonyl, C₃-C₈-cycloalkylsulphonyl, aryl-C₀-C₈-alkylsulphonyl, heterocyclylsulphonyl, C₃-C₁₂-cycloalkyl-C₁-C₈-alkanoyl, C₃-C₁₂-cycloalkyl-C₃-C₈-cycloalkanoyl, aryl-C₁-C₈-alkanoyl, aryl-C₃-C₈-cycloalkanoyl, C₁-C₈-alkanoyl, C₁-C₈-alkoxycarbonyl, optionally N-mono- or N,N-di-C₁-C₈-alkylated carbamoyl-C₀-C₈-alkyl, aryl-C₀-C₄-alkyl or heterocyclyl-C₀-C₄-alkyl, which radicals may be substituted by 1-4 C₁-C₈-alkyl, C₃-C₈-cycloalkyl, C₃-C₈-cycloalkoxy, amino, C₁₋₆-alkylamino, di-C₁₋₆-alkylamino, C₀-C₆-alkylcarbonylamino, halogen, cyano, hydroxyl, oxide, oxo, trifluoromethyl, C₁-C₈-alkoxy, optionally N-mono- or N,N-di-C₁-C₈-alkylated carbamoyl, C₁-C₈-alkoxycarbonyl C₁₋₆-alkylene-dioxy, aryl or heterocyclyl; or
is b) together with R₁ and the nitrogen atom to which they are bonded, a saturated or partly unsaturated 4-8-membered heterocyclic ring which may contain an additional nitrogen, oxygen or sulphur atom or an -SO- or -SO2- group, in which case the additional nitrogen atom may optionally be substituted by C₁-C₈-alkyl, C₁-C₈-alkanoyl, C₁-C₈-alkoxycarbonyl, aryl or heterocyclyl radicals, and this heterocyclic ring may be part of a bicyclic or tricyclic ring system having a total of up to 16 members, and the second ring may also contain a nitrogen, oxygen or sulphur atom or an -SO- or -SO2- group, and the nitrogen atom of the second ring may optionally be substituted by C₁-C₈-alkyl, C₁-C₈-alkanoyl, C₁-C₈-alkoxycarbonyl, aryl or heterocyclyl radicals and all ring systems mentioned may be substituted by 1-4 C₁-C₈-alkyl, halogen, hydroxyl, oxide, oxo, cyano, trifluoromethyl, C₁-C₈-alkoxy, C₁-C₈-alkoxy-C₁-C₈-alkyl, C₁-C₈-alkoxy-C₁-C₈-alkoxy, C₁-C₈-alkoxycarbonylamino, C₁-C₈-alkylcarbonylamino, C₁-C₈-alkylamino, N,N-di-C₁-C₈-alkylamino, aryl-C₀-C₄-alkyl, aryloxy-C₀-C₄-alkyl, aryl-C₀-C₄-alkyl-C₁-C₈-alkoxy, aryloxy-C₀-C₄-alkyl-C₁-C₈-alkoxy, heterocyclyl-C₀-C₄-alkyl, heterocyclyloxy-C₀-C₄-alkyl, heterocyclyl-C₀-C₄-alkyl-C₁-C₈-alkoxy or heterocyclyloxy-C₀-C₄-alkyl-C₁-C₈-alkoxy;
R₃ is hydrogen, C₁-C₈-alkyl, C₁-C₈-alkoxycarbonyl or C₁-C₈-alkanoyl;
R₄ is hydrogen, C₁-C₈-alkyl, C₁-C₈-alkoxycarbonyl or C₁-C₈-alkanoyl;
R₅ are each independently hydrogen or C₁-C₈-alkyl, or, together with the carbon atom to which they are bonded, are a C₃-C₈-cycloalkylidene radical;
R is an optionally substituted unsaturated carbocyclic or heterocyclic radical; one of the X₁ and X₂ radicals is carbonyl and the other is methylene; and salts thereof.

Possible substituents for R are aliphatic, araliphatic, heterocycloaliphatic-aliphatic or heteroaliphatic radicals, optionally esterified or amidated carboxyl, optionally aliphatically or heteroaliphatically substituted amino or else optionally aliphatically, araliphatically, heterocycloaliphatic-aliphatically or heteroarylaliphatically etherified hydroxyl.

In the context of the above definitions, the above general terms preferably have the following definitions:

Aliphatic radicals are, for example, lower alkyl, hydroxy(lower alkyl), (lower alkanoyl)oxy (lower alkyl), (lower alkoxy)(lower alkyl), (lower alkoxy)(lower alkoxy)(lower alkyl), an optionally amidated carboxyl or carboxy(lower alkyl) group, optionally esterified or amidated dicarboxy(lower alkyl), optionally esterified or amidated carboxy(hydroxy)(lower alkyl), (lower alkane)sulphonyl(lower alkyl) or optionally N-mono- or N,N-di(lower alkyl)ated sulphamoyl(lower alkyl).

Optionally aliphatically, araliphatically, heterocycloaliphatic-aliphatically or heteroarylaliphatically etherified hydroxyl is, for example, hydroxyl, lower alkoxy, hydroxy(lower alkoxy), (lower alkanoyl)oxy(lower alkoxy), (lower alkoxy)(lower alkoxy), (lower alkoxy)(lower alkoxy)-(lower alkoxy), polyhalo(lower alkoxy), cyano(lower alkoxy), an amino(lower alkoxy) radical which is optionally N-(lower alkanoyl)ated, N-mono- or N,N-di(lower alkyl)ated, or is N,N-disubstituted by lower alkylene, hydroxy-, (lower alkoxy)- or (lower alkoxy)(lower alkoxy)-(lower alkylene), or by optionally N'-(lower alkanoyl)ated aza(lower alkylene), oxa(lower alkylene) or optionally S-oxidized thia(lower alkylene), each of which is optionally N-(lower alkanoyl)ated or is N'-substituted or N'-(lower alkyl)ated by (lower alkoxy)carbonyl or (lower alkoxy)(lower alkyl), optionally substituted phenyl- or pyridyl(lower alkoxy), optionally amidated caboxyl or carboxy(lower alkoxy) or tetrazolyl(lower alkoxy).

Heteroaliphatic radicals are, for example, optionally N-(lower alkanoyl)ated, N-mono- or N,N-di(lower alkyl)ated, or amino(lower alkyl) radicals N,N-disubstituted by lower alkylene, hydroxy-, (lower alkoxy)- or (lower alkoxy)(lower alkoxy)(lower alkylene), or by aza(lower alkylene), oxa(lower alkylene) or optionally S-oxidized thia(lower alkylene), each of which is optionally N'-(lower alkanoyl)ated or is N'-substituted or N'-(lower alkyl)ated by (lower alkoxy)carbonyl or (lower alkoxy)(lower alkyl), or N-mono- or N,N-di(lower alkyl)ated thiocarbamoyl(lower alkyl) radicals.

Araliphatic or heteroarylaliphatic radicals are, for example, optionally substituted phenyl- or pyridinyl(lower alkyl) groups.

Cycloaliphatic-aliphatic radicals are, for example, cycloalkyl(lower alkyl) or optionally esterified or amidated carboxycycloalkyl(lower alkyl).

Optionally aliphatically substituted amino is, for example, optionally N-(lower alkanoyl)ated or N-mono- or N,N-di(lower alkyl)ated amino.

Optionally heteroaliphatically substituted amino is, for example, amino which is optionally N-(lower alkanoyl)ated, N-mono- or N,N-di(lower alkyl)ated, or N,N-disubstituted by lower alkylene, hydroxy-, (lower alkoxy)-, (lower alkoxy)carbonyl- or (lower alkoxy)(lower alkoxy)-(lower alkylene), or by optionally N'-(lower alkanoyl)ated aza(lower alkylene), oxa(lower alkylene) or optionally S-oxidized thia(lower alkylene), each of which is optionally N'-substituted or N'-(lower alkyl)ated by (lower alkoxy)carbonyl or (lower alkoxy)(lower alkyl).

Optionally esterified or amidated carboxyl is, for example, optionally aliphatically or araliphatically etherified carboxyl or aliphatically substituted carbamoyl.

Heterocyclyl bonded via a ring carbon or ring nitrogen atom contains generally from 4 to 8, in particular from 5 to 7, ring atoms, and may have 1 or 2 fused-on phenyl or cycloalkyl radicals, or else be present as a spiro compound. Examples include pyrrolidino, piperidino, piperazino, morpholino, thiomorpholino, tetrahydrofuranyl, furanyl, pyranyl, tetrahydropyranyl, thiazolyl, oxazolyl, imidazolyl, indolinyl, isoindolinyl, 2,3-dihydrobenzimidazolyl, 1,2,3,4-tetrahydroquinolyl, 1,2,3,4-tetrahydroisoquinolyl, 1,2,3,4-tetrahydro-1,3-benzodiazinyl, 1,2,3,4-tetrahydro-1,4-benzodiazinyl, 3,4-dihydro-2H-1,4-benzoxazinyl, 3,4-dihydro-2H-1,4-benzothiazinyl, 3,4-dihydro-2H-1,3-benzothiazinyl, 3,4,5,6,7,8-hexahydro-2H-1,4-benzoxazinyl, 3,4,5,6,7,8-hexahydro-2H-1,4-benzothiazinyl, 2,3,4,5-tetrahydro-1H-1-benz[6,7-b]azepinyl and 5,6-dihydrophenanthridinyl. The radicals mentioned may be unsubstituted or N-substituted and/or C-substituted, in which case in particular 1, 2 or 3 substituents may be present.

Aryl contains generally 6-14, preferably 6-10 carbon atoms and is, for example, phenyl, indenyl, e.g. 2- or 4-indenyl, or naphthyl, e.g. 1- or 2-naphthyl. Preference is given to aryl having 6 -10 carbon atoms, in particular phenyl or 1- or 2-naphthyl. The radicals mentioned may be unsubstituted or mono- or polysubstituted, e.g. mono- or disubstituted, for example by C₁-C₈-alkyl, cyano, hydroxyl, amino, C₁₋₆-alkylamino, di-C₁₋₆-alkylamino, C₂₋₆-alkenyl, C₂₋₆-alkynyl, C₀₋₆-alkylcarbonylamino, C₁₋₈-alkoxycarbonylamino, halogen, oxo, trifluoromethyl, C₁-C₈-alkoxy, optionally N-mono- or N-di-C₁-C₈-alkylcarbamoyl, C₁-C₈-alkoxycarbonyl C₁₋₆-alkylenedioxy, aryl or heterocyclyl, and the substituents may be in any position, for example in the o-, m- or p-position of the phenyl radical, or in the 3- or 4-position of the 1- or 2-naphthyl radical, and a plurality of identical or different substituents may also be present.

Useful substituents of phenyl, phenyl(lower alkoxy), pyridyl(lower alkyl), pyridyl(lower alkoxy) and optionally hydrogenated and/or oxo-substituted heteroaryl or heterocyclyl include, for example, lower alkyl, lower alkoxy, hydroxyl, cyano, oxide, oxo, nitro, amino, (lower alkyl)amino, di(lower alkyl)amino, halogen, optionally N-mono- or N-di-C₁-C₈-alkylcarbamoyl, C₁-C₈-alkoxycarbonyl, C₁₋₆-alkylenedioxy and trifluoromethyl, and up to 3, in particular 1 or 2, identical or different substituents of those mentioned may be present.

Optionally hydrogenated and/or oxo-substituted heteroaryl radicals are, for example, optionally partially hydrogenated and/or benzofused 5-membered aza-, diaza-, triaza-, oxadiaza- or tetraazaaryl or 6-membered aza- or diazaaryl radicals, such as optionally oxo-substituted pyrrolidinyl, e.g. pyrrolidinyl or oxopyrrolidinyl, imidazolyl, e.g. imidazol-4-yl, benzimidazolyl, e.g. benzimidazol-2-yl, oxadiazolyl, e.g. 1,2,4-oxadiazol-5-yl, pyridyl, e.g. pyridin-2-yl, oxopiperidinyl, dioxopiperidinyl, oxothiazolyl, oxooxazolinyl or quinolinyl, e.g. quinolin-2-yl, or optionally N-(Iower alkanoyl)ated piperidyl, such as 1-(lower alkanoyl)-piperidinyl.

Lower alkyl substituted by a heteroaryl radical which is bonded via a carbon atom and is optionally hydrogenated and/or oxo-substituted has, as the optionally hydrogenated heteroaryl radical, for example, an optionally partially hydrogenated and/or benzofused 5-membered aza-, diaza-, triaza-, oxadiaza- or tetraazaaryl radical, or 6-membered aza- or diazaaryl radical, and is, for example, optionally oxo-substituted pyrrolidinyl(lower alkyl), e.g. pyrrolidinyl(lower alkyl) or oxopyrrolidinyl(lower alkyl), imidazolyl(lower alkyl), benzimidazolyl(lower alkyl), oxadiazolyl(lower alkyl), pyridyl(lower alkyl), oxopiperidinyl(lower alkyl), dioxopiperidinyl(lower alkyl), oxothiazolyl(lower alkyl), oxooxazolinyl(lower alkyl) or quinolinyl(lower alkyl), and equally morpholinocarbonyl(lower alkyl) or optionally N-(lower alkanoyl)ated piperidyl(lower alkyl), such as 1-(lower alkanoyl)piperidinyl(lower alkyl).

Lower alkyl, for example C₁-C₈-alkyl, may be straight-chain or branched and is, for example, methyl, ethyl, propyl, isopropyl, butyl, isobutyl, seo-butyl, tert-butyl, or a pentyl, hexyl or heptyl group.

Lower alkenyl, for example C₂-C₈-alkenyl, may be straight-chain or branched and is, for example, vinyl, allyl, propenyl, isopropenyl, butenyl, isobutenyl or a pentenyl, hexenyl or heptenyl group.

Lower alkynyl, for example C₂-C₆-alkynyl, may be straight-chain or branched and is, for example, ethynyl, 1-propynyl, 3-propynyl, 1-butynyl, 3-butynyl, 4-butynyl, or a pentynyl or hexynyl group.

C₁₋₆-Alkylenedioxy is, for example, methylenedioxy, ethylenedioxy, 1,3-propylenedioxy or 1,2-propylenedioxy.

Amino(lower alkoxy) is, for example, amino-C₁-C₄-alkoxy, such as 2-aminoethoxy, 3-aminopropyloxy, 4-aminobutyloxy or 5-aminopentyloxy.

Aryl-C₁-C₈-alkanoyl is one of the aryl radicals mentioned which is bonded to the rest of the compound via a C₁-C₈-alkanoyl group, for example phenylformyl, phenylacetyl, 3-phenylpropionyl, 2-phenyl-2-methylpropionyl or phenylpivaloyl.

Aryl-C₀-C₄-alkyl is one of the aryl radicals mentioned which is bonded to the rest of the compound either directly or a via a C₁-C₄-alkyl group.

Aryl-C₃-C₈-cycloalkanoyl is one of the aryl radicals mentioned which is bonded to the rest of the compound via a C₃-C₈-cycloalkanoyl group, for example 1-phenylcyclobutanoyl.

Carbamoyl(lower alkoxy) is, for example, carbamoyl-C₁-C₈-alkoxy, such as carbamoyl-methoxy, 2-carbamoylethoxy, 3-carbamoylpropyloxy 2-(3-carbamoyl)propyloxy, 2-carbamoylpropyloxy, 3-(1-carbamoyl)propyloxy, 2-(2-carbamoyl)propyloxy, 2-(carbamoyl-2-methyl)-propyloxy, 4-carbamoylbutyloxy, 1-carbamoylbutyloxy, 1-(1-carbamoyl-2-methyl)butyloxy, 3-(4-carbamoyl-2-methyl)butyloxy, in particular 3-carbamoylpropyloxy or 2-carbamoyl-2-methylethoxy.

Carbamoyl(lower alkyl) is, for example, carbamoyl-C₁-C₈-alkyl, such as carbamoylmethyl, 2-carbamoylethyl, 3-carbamoylpropyl, 2-(3-carbamoyl)propyl, 2-carbamoylpropyl, 3-(1-carbamoyl)propyl, 2-(2-carbamoyl)propyl, 2-(carbamoyl-2-methyl)propyl, 4-carbamoyl-butyl, 1-carbamoyl butyl, 1-(1-carbamoyl-2-methyl)butyl, 3-(4-carbamoyl-2-methyl)butyl, in particular 3-carbamoylpropyl or 2-carbamoyl-2-methylethyl.

Carboxy(lower alkoxy) is, for example, carboxy-C₁-C₄-alkoxy, such as carboxymethoxy, 2-carboxyethoxy, 2- or 3-carboxypropyloxy 2-carboxy-2-methylpropyloxy, 2-carboxy-2-ethyl-butyloxy or 4-carboxybutyloxy, in particular carboxymethoxy.

Cycloalkoxy has, for example, from 3 to 8, in particular from 5 to 7 ring members, and is, for example, cyclopentyloxy, cyclohexyloxy or cycloheptyloxy, and also cyclopropyloxy, cyclobutyloxy or cyclooctyloxy.

Cycloalkyl has, for example, from 3 to 12, in particular from 5 to 10 ring members, and is, for example, cyclopentyl, cyclohexyl or cycloheptyl, and also cyclopropyl, cyclobutyl, cyclooctyl or adamantyl.

C₃-C₁₂-Cycloalkyl-C₃-C₈-cycloalkanoyl is one of the cycloalkyl radicals mentioned which is bonded to the rest of the compound via a C₃-C₈-cycloalkanoyl group, for example 1-cyclohexylcyclobutanoyl.

C₃-C₁₂-Cycloalkyl-C₁-C₈-alkanoyl is one of the cycloalkyl radicals mentioned which is bonded to the rest of the compound via a C₁-C₈-alkanoyl group, for example adamantylformyl, cyclobutylformyl, cyclopentylformyl, cyclohexylformyl, cyclohexylacetyl, 2-cyclopentyl-2-methylpropionyl, 2-cyclohexylpropionyl, 3-cyclohexylpropionyl or 2-cyclohexyl-2-methylpropionyl.

Di(lower alkyl)amino(lower alkoxy) is, for example, N,N-di-C₁-C₄-alkylamino-C₁-C₄-alkoxy, such as 2-dimethylaminoethoxy, 3-dimethylaminopropyloxy, 4-dimethylaminobutyloxy, 2-diethylaminoethoxy, 2-(N-methyl-N-ethylamino)ethoxy or 2-(N-butyl-N-methylamino)ethoxy, in particular 3-dimethylaminopropyloxy.

Di(lower alkyl)amino(lower alkyl) is, for example, N,N-di-C₁-C₄-alkylamino-C₁-C₄-alkyl, such as 2-dimethylaminoethyl, 3-dimethylaminopropyl, 4-dimethylaminobutyl, 2-diethylaminoethyl, 2-(N-methyl-N-ethylamino)ethyl or 2-(N-butyl-N-methylamino)ethyl, in particular dimethylaminomethyl.

Di(lower alkyl)amino is, for example, di-C₁-C₄-alkylamino, such as dimethylamino, N-methyl-N-ethylamino, diethylamino, N-methyl-N-propylamino or N-butyl-N-methylamino.

Di(lower alkyl)amino(lower alkoxy) is, for example, N,N-di-C₁-C₄-alkylamino-C₁-C₄-alkoxy, such as 2-dimethylaminoethoxy, 3-dimethylaminopropyloxy, 2-dimethylaminopropyloxy, 2-(dimethylamino-2-methyl)propyloxy or 2-(1-dimethylamino-3-methyl)butyloxy, in particular 3-dimethylaminopropyloxy.

Di(lower alkyl)carbamoyl is, for example, di-C₁-C₄-alkylcarbamoyl, such as dimethylcarbamoyl, N-methyl-N-ethylcarbamoyl, diethylcarbamoyl, N-methyl-N-propylcarbamoyl or N-butyl-N-methylcarbamoyl.

Di(lower alkyl)carbamoyl(lower alkyl) is, for example, N,N-di-C₁-C₄-alkylcarbamoyl-C₁-C₄-alkyl, such as 2-dimethylcarbamoylethyl, 3-dimethylcarbamoylpropyl, 2-dimethylcarbamoylpropyl, 2-(dimethylcarbamoyl-2-methyl)propyl or 2-(1-dimethylcarbamoyl-3-methyl)butyl, in particular 2-dimethylcarbamoylethyl.

Halogen is, for example, fluorine, chlorine, bromine or iodine, preferably fluorine and chlorine.

Heterocyclyl-C₀-C₄-alkyl is one of the heterocyclyl radicals mentioned which is bonded to the rest of the compound either directly or via a C₁-C₄-alkyl group.

Morpholino(lower alkoxy) is, for example, morpholino-C₁-C₄-alkoxy, such as morpholino-methoxy, 2-morpholinoethoxy, 3-morpholinopropyloxy or 4-morpholinobutyloxy, in particular 2-morpholinoethoxy or 3-morpholinopropyloxy.

Morpholino(lower alkyl) is, for example, morpholino-C₁-C₄-alkyl, such as morpholinomethyl, 2-morpholinoethyl, 3-morpholinopropyl or 4-morpholinobutyl, in particular morpholinomethyl, 2-morpholinoethyl or 3-morpholinopropyl.

Morpholino(lower alkyl)carbamoyl(lower alkoxy) is, for example, N-(morpholino-C₁-C₄-alkylcarbamoyl)-C₁-C₄-alkoxy, in particular 2-morpholinoethylcarbamoylmethoxy.

Lower alkanoyl is, for example, C₁-C₈-alkanoyl, such as formyl, acetyl or pivaloyl.

(Lower alkanoyl)piperazino(lower alkyl) is, for example, N'-C₂-C₈-(lower alkanoyl)piperazino-C₁-C₄-alkyl, such as 4-acetylpiperazinomethyl.

Lower alkoxy is, for example, C₁-C₈-alkoxy, preferably C₁-C₄-alkoxy, such as methoxy, ethoxy, propyloxy, isopropyloxy, butyloxy, isobutyloxy, sec-butyloxy, tert-butyloxy, pentyloxy, or a hexyloxy or heptyloxy group.

Lower alkoxycarbonyl is, for example, C₁-C₄-alkoxycarbonyl, such as methoxycarbonyl, ethoxycarbonyl, propyloxycarbonyl, isopropyloxycarbonyl, butyloxycarbonyl, isobutyloxycarbonyl, sec-butyloxycarbonyl, tert-butyloxycarbonyl, pentyloxycarbonyl, or a hexyloxycarbonyl or heptyloxycarbonyl group.

(Lower alkoxy)(lower alkoxy) is, for example, C₁-C₄-alkoxy-C₂-C₄-alkoxy, such as 2-methoxy-, 2-ethoxy- or 2-propyloxyethoxy, 3-methoxy- or 3-ethoxypropyloxy or 4-methoxybutyloxy, in particular 2-methoxyethoxy, 3-methoxypropyloxy, 4-methoxybutyloxy, 5-methoxypentyloxy.

(Lower alkoxy)(lower alkoxy)(lower alkoxy) is, for example, C₁-C₄-alkoxy-C₁-C₄-alkoxy-C₁-C₄-alkoxy, such as 2-methoxy-, 2-ethoxy- or 2-propyloxyethoxymethoxy, 2-(2-methoxy-, 2-ethoxy- or 2-propyloxyethoxy)ethoxy, 3-(3-methoxy- or 3-ethoxypropyloxy)propyloxy or 4-(2-methoxybutyloxy)butyloxy, in particular 2-(methoxymethoxy)ethoxy or 2-(2-methoxyethoxy)ethoxy.

(Lower alkoxy)(lower alkyl) is, for example, C₁-C₄-alkoxy-C₁-C₄-alkyl, such as ethoxymethyl, propyloxymethyl, butyloxymethyl, 2-methoxy-, 2-ethoxy- or 2-propyloxyethyl, 3-methoxy- or 3-ethoxypropyl or 4-methoxybutyl, in particular 3-methoxypropyl or 4-methoxybutyl, in particular propyloxymethyl.

Lower alkyl may be straight-chain or branched and/or bridged and is, for example, corresponding C₁-C₈-alkyl, such as methyl, ethyl, propyl, isopropyl, butyl, isobutyl, sec-butyl, tert-butyl, or a pentyl, hexyl, heptyl or octyl group.

(Lower alkyl)amino is, for example, C₁-C₄-alkylamino, such as methylamino, ethylamino, propylamino, butylamino, isobutylamino, sec-butylamino or tert-butylamino.

(Lower alkyl)carbamoyl is, for example, C₁-C₄-alkylcarbamoyl, such as methylcarbamoyl, ethylcarbamoyl, propylcarbamoyl, butylcarbamoyl, isobutylcarbamoyl, sec-butylcarbamoyl or tert-butylcarbamoyl, in particular methylcarbamoyl.

(Lower alkyl)carbamoyl(lower alkoxy) is, for example, N-C₁-C₄-alkylcarbamoyl-C₁-C₄-alkoxy, such as 2-propylcarbamoylethoxy, 3-ethylcarbamoylpropyloxy, 2-ethylcarbamoylpropyloxy, 2-(methylcarbamoyl-2-methyl)propyloxy, 2-(1-methylcarbamoyl-3-methyl)butyloxy or in particular butylcarbamoylmethoxy.

(Lower alkyl)carbamoyl(lower alkyl) is, for example, N-C₁-C₈-alkylcarbamoyl-C₁-C₄-alkyl, such as methyl- or dimethylcarbamoyl-C₁-C₄-alkyl, e.g. methylcarbamoylmethyl, 2-methyl-carbamoylethyl, 3-methylcarbamoylpropyl or in particular 2-methylcarbamoyl-2-methylpropyl.

(Lower alkyl)piperazino(lower alkyl) is, for example, N'-C₁-C₄-alkylpiperazino-C₁-C₄-alkyl, such as N'-methylpiperazinomethyl, 2-(N'-methylpiperazino)ethyl, 3-(N'-methylpiperazino)-propyl or 4-(N'-methylpiperazino)butyl, in particular N'-methylpiperazinomethyl.

Piperidino(lower alkoxy) is, for example, piperidino-C₁-C₄-alkoxy, such as 2-piperidinoethoxy, 3-piperidinopropyloxy or 4-piperidinobutyloxy, in particular 2-piperidinoethoxy.

Piperidino(lower alkyl) is, for example, piperidino-C₁-C₄-alkyl, such as piperidinomethyl, 2-piperidinoethyl, 3-piperidinopropyl or 4-piperidinobutyl, in particular piperidinomethyl.

Pyrrolidino(lower alkyl) is, for example, pyrrolidino-C₁-C₄-alkyl, such as pyrrolidinomethyl, 2-pyrrolidinoethyl, 3-pyrrolidinopropyl or 4-pyrrolidinobutyl, in particular pyrrolidinomethyl.

C₁-C₈-Alkylsulphonyl is, for example, methylsulphonyl, ethylsulphonyl, propylsulphonyl, isopropylsulphonyl, butylsulphonyl, isobutylsulphonyl, sec-butylsulphonyl, tert-butylsulphonyl, or a pentyl, hexyl, heptyl or octylsulphonyl group.

C₃-C₈-Cycloalkylsulphonyl is, for example, cyclopentylsulphonyl, cyclohexylsulphonyl or cycloheptylsulphonyl, and also cyclopropylsulphonyl, cyclobutylsulphonyl or cyclooctylsulphonyl.

Aryi-C₀-C₈-alkylsulphonyl is one of the aryl radicals mentioned which is bonded to the rest of the compound either via a sulphonyl group or via a C₁-C₈-alkylsulphonyl group, for example phenylsulphonyl, benzylsulphonyl or phenyldimethylenesulphonyl.

Heterocyclylsulphonyl is one of the heterocyclyl radicals mentioned which is bonded to the rest of the compound via a sulphonyl group.

Depending on the presence of asymmetric carbon atoms, the inventive compounds may be present in the form of isomer mixtures, especially as racemates, or in the form of pure isomers, especially of optical antipodes.

Salts of compounds having salt-forming groups are in particular acid addition salts, salts with bases, or, in the presence of a plurality of salt-forming groups, in some cases also mixed salts or internal salts.

Salts are primarily the pharmaceutically usable or nontoxic salts of compounds of the formula I.

Such salts are formed, for example, from compounds of the formula I with an acidic group, for example a carboxyl or sulpho group, and are, for example, the salts thereof with suitable bases such as non-toxic metal salts derived from metals of group la, Ib, Ila and Ilb of the Periodic Table of the Elements, for example alkali metal, in particular lithium, sodium, or potassium, salts, alkaline earth metal salts, for example magnesium or calcium salts, and also zinc salts and ammonium salts, including those salts which are formed with organic amines, such as optionally hydroxy-substituted mono-, di- or trialkylamines, in particular mono-, di- or tri(lower alkyl)amines, or with quaternary ammonium bases, e.g. methyl-, ethyl-, diethyl- or triethylamine, mono-, bis- or tris(2-hydroxy(lower alkyl))amines, such as ethanol-, diethanol- or triethanolamine, tris(hydroxymethyl)methylamine or 2-hydroxy-tert-butylamine, N,N-di(lower alkyl)-N-(hydroxy(lower alkyl))amine, such as N,N-di-N-dimethyl-N-(2-hydroxyethyl)amine, or N-methyl-D-glucamine, or quaternary ammonium hydroxides such as tetrabutylammonium hydroxide. The compounds of the formula I having a basic group, for example an amino group, may form acid addition salts, for example with suitable inorganic acids, e.g. hydrohalic acid such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic, sulpho or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, and also amino acids, for example the alpha-amino acids mentioned above, and also methanesulphonic acid, ethanesulphonic acid, 2-hydroxyethanesulphonic acid, ethane-1,2-disulphonic acid, benzenesulphonic acid, 4-methylbenzenesulphonic acid, naphthalene-2-sulphonic acid, 2- or 3-phosphoglycerate, glucose 6-phosphate, N-cyclohexylsulphamic acid (with formation of the cyclamates) or with other acidic organic compounds such as ascorbic acid. Compounds of the formula I having acidic and basic groups may also form internal salts.

For the isolation and purification, pharmaceutically unsuitable salts may also find use.

The compound groups mentioned hereinbelow are not to be regarded as closed, but rather it is possible in a sensible manner, for example, to replace general by more specific definitions by exchanging parts of these compound groups with one another or with the definitions given above, or omitting them.

The invention relates primarily to the use of compounds of the formula I, in particular of the formula la 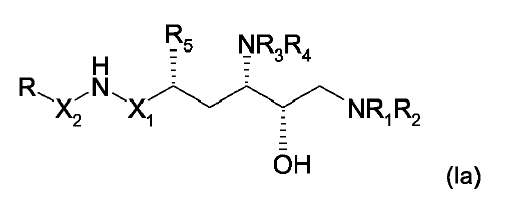 where
R₁ is a) hydrogen; or
is b) C₁-C₈-alkyl or C₃-C₈-cycloalkyl;
R₂ is a) C₁-C₈-alkyl, C₃-C₈-cycloalkyl, C₁-C₈-alkanoyl, heterocyclyl-C₁-C₈-alkanoyl, C₃-C₁₂-cycloalkyl-C₁-C₈-alkanoyl or aryl-C₁-C₈-alkanoyl, which radicals may be substituted by 1-4 C₁-C₈-alkyl, C₃-C₈-cycloalkyl, C₃-C₈-cycloalkoxy, C₁₋₆-alkylamino, cyano, halogen, hydroxyl, oxide, C₀-C₆-alkylcarbonylamino, C₁-C₆-alkoxycarbonylamino, C₁-C₈-alkoxy, oxo, trifluoromethyl or aryl; or
is b) together with R₁ and the nitrogen atom to which they are bonded, a saturated or partly unsaturated, 4-8-membered, heterocyclic ring which may contain an additional nitrogen or oxygen atom, in which case the additional nitrogen atom may optionally be substituted by C₁-C₈-alkyl or C₁-C₈-alkanoyl, and this heterocyclic ring may be part of a bicyclic or tricyclic ring system having a total of up to 16 members and the second ring may also contain a nitrogen or oxygen atom, and the nitrogen atom of the second ring may optionally be substituted by C₁-C₈-alkyl or C₁-C₈-alkanoyl, and all ring systems mentioned may be substituted by 1-4 C₁-C₈-alkyl, hydroxyl, oxo, oxide, C₁-C₈-alkoxy, C₁-C₈-alkoxy-C₁-C₈-al koxy, C₁-C₈-alkylcarbonylamino or aryloxy-C₀-C₄-alkyl-C₁-C₈-alkoxy;
R₃ and R₄ are each hydrogen,
R₅ is C₁-C₄-alkyl, such as methyl or isopropyl,
R is a 2-R_{A}-4-R_{c}-phenyl radical, 2-R_{A}-pyridin-3-yl radical or 3-R_{A}-pyridin-2-yl radical,
where
R_{A} is C₁-C₄-alkoxy-C₁-C₄-alkyl such as propyloxymethyl, morpholino-C₁-C₄-alkyl such as 2-morpholinoethyl or 3-morpholinopropyl, C₁-C₈-alkanoylpiperazino-C₁-C₄-alkyl such as N'-acetylpiperazinomethyl, C₁-C₈-alkoxy such as propyloxy, C₁-C₄-alkoxy-C₁-C₅-alkoxy such as 2-methoxyethoxy, 3-methoxypropyloxy, 4-methoxy-butyloxy or 5-methoxypentyloxy, C₁-C₄-alkoxy-C₂-C₄-alkenyloxy such as 4-methoxy-but-2-enyloxy, C₁-C₄-alkoxy-C₁-C₄-alkoxy-C₁-C₄-alkoxy such as 2-(methoxy-methoxy)ethoxy or 2-(2-methoxyethoxy)ethoxy, amino-C₁-C₄-alkoxy such as 2-aminoethoxy or 3-aminopropyloxy, di-C₁-C₄-alkylamino-C₁-C₄-alkoxy such as 3-dimethylaminopropyloxy, C₁-C₈-alkanoyi-amino-C₁-C₄-alkoxy such as N-acetylaminoethoxy, C₁-C₈-alkanoyl-amino-C₁-C₄-alkyl such as N-acetylaminoethyl, carbamoyl-C₁-C₄-alkoxy such as 2-carbamoylethoxy or carbamoyl, and
R_{c} is hydrogen, di-C₁-C₄-alkylamino-C₁-C₄-alkyl such as dimethylaminomethyl, piperidino-C₁-C₄-alkyl such as piperidinomethyl, pyrrolidino-C₁-C₄-alkyl such as pyrrolidinomethyl, morpholino-C₁-C₄-alkyl such as morpholinomethyl, C₁-C₈-alkanoylpiperazino-C₁-C₄-alkyl such as N'-acetylpiperazinomethyl, or C₁-C₄-alkylpiperazino-C₁-C₄-alkyl such as N'-methylpiperazinomethyl, morpholino, C₁-C₄-alkoxy such as methoxy, morpholino-C₁-C₄-alkoxy such as 2-morpholinoethoxy or 3-morpholinopropyloxy, morpholino-C₁-C₄-alkylcarbamoyl-C₁-C₄-alkoxy such as 2-morpholinoethylcarbamoylmethoxy, piperidino-C₁-C₄-alkoxy such as 2-piperidinoethoxy, carboxyl, carbamoyl, C₁-C₄-alkylcarbamoyl such as methylcarbamoyl, carboxy-C₁-C₄-alkoxy such as carboxymethoxy, di-C₁-C₄-alkylamino-C₁-C₄-alkoxy, such as 3-dimethylaminopropyloxy, C₁-C₈-alkylcarbamoyl-C₁-C₄-alkoxy such as butylcarbamoylmethoxy, or tetrazolyl-C₁-C₄-alkoxy, such as tetrazol-5-ylmethoxy,
X₁ is methylene and X₂ is carbonyl,
and salts thereof, in particular pharmaceutically usable salts thereof.

The invention relates specifically to the use of the compounds of the formula I specified in the examples and salts thereof, in particular the pharmaceutically usable salts thereof.

Salts obtained may be converted to other salts in a manner known per se, acid addition salts, for example, by treating with a suitable metal salt such as a sodium, barium or silver salt, of another acid in a suitable solvent in which an inorganic salt which forms is insoluble and thus separates out of the reaction equilibrium, and base salts by release of the free acid and salt reformation.

The compounds of the formula I, including their salts, may also be obtained in the form of hydrates or include the solvent used for the crystallization.

Owing to the narrow relationship between the novel compounds in free form and in the form of their salts, the free compounds and salts thereof refer above and below analogously and appropriately, where appropriate, also to the corresponding salts and free compounds thereof respectively.

Stereoisomer mixtures, i.e. mixtures of diastereomers and/or enantiomers, for example racemic mixtures, may be separated into the corresponding isomers in a manner known per se by suitable separation processes. For instance, diastereomer mixtures may be separated into the individual diastereomers by fractional crystallization, chromatography, solvent partition, etc. After conversion of the optical antipodes to diastereomers, for example by reacting with optically active compounds, e.g. optically active acids or bases, racemates may be separated from one another by chromatography on column materials laden with optically active compounds or by enzymatic methods, for example by selective conversion of only one of the two enantiomers. This separation may be effected either at the stage of one of the starting materials or on the compounds of the formula I. It is possible for the configuration at individual chiral centres in a compound of the formula I to be inverted selectively.

For example, the configuration of asymmetric carbon atoms which bear nucleophilic substituents, such as amino or hydroxyl, may be inverted by second-order nucleophilic substitution, if appropriate after conversion of the bonded nucleophilic substituent to a suitable nucleofugic leaving group and reaction with a reagent which introduces the original substituents, or the configuration at carbon atoms having hydroxyl groups can be inverted by oxidation and reduction, analogously to the process in the European patent application EP-A-0 236 734.

Also advantageous is the reactive functional modification of the hydroxyl group and subsequent replacement thereof by hydroxyl with inversion of configuration. To this end, the amino and hydroxyl group drawn in formula I are bridged by a bivalent group, in particular carbonyl, to obtain a compound which can be cleaved again on treatment with thionyl chloride with inversion of configuration.

The compounds of the formula (I) may be prepared in an analogous manner to the preparation processes known from the literature. The starting materials for carrying out the preparation processes are described, for example, in EP 0716077. The inventive compounds of the formula I and salts of such compounds having at least one salt-forming group are obtained by processes known per se, as also described on pages 13 to 16 of WO2005/070871 which are herewith incorporated.

Details of specific preparation variants can be taken from the examples.

The compounds of the formula (I) may also be prepared in optically pure form. The separation into antipodes may be effected by methods known per se, either preferably at a synthetically early stage by salt formation with an optically active acid, for example (+)- or (-)-mandelic acid, and separation of the diastereomeric salts by fractional crystallization, or preferably at a rather later stage by derivatization with a chiral auxiliary building block, for example (+)- or (-)-camphanoyl chloride, and separation of the diastereomeric products by chromatography and/or crystallization and subsequent cleavage of the bond to the chiral auxiliary. The pure diastereomeric salts and derivatives may be analysed with common spectroscopic methods to determine the absolute configuration of the piperidine present, and X-ray spectroscopy on single crystals constitutes a particularly suitable method.

Prodrug derivatives of the compounds described in the present context are derivatives thereof which, on *in vivo* application, release the original compound by a chemical or physiological process. A prodrug may be converted to the original compound, for example, when a physiological pH is attained or by enzymatic conversion. Prodrug derivatives may, for example, be esters of freely available carboxylic acids, S- and O-acyl derivatives of thiols, alcohols or phenols, and the acyl group is as defined in the present context. Preference is given to pharmaceutically usable ester derivatives which are converted by solvolysis in physiological medium to the original carboxylic acid, for example lower alkyl esters, cycloalkyl esters, lower alkenyl esters, benzyl esters, mono- or disubstituted lower alkyl esters such as lower ω-(amino, mono- or dialkylamino, carboxyl, lower alkoxycarbonyl)-alkyl esters or such as lower α-(alkanoyloxy, alkoxycarbonyl or dialkylaminocarbonyl)-alkyl esters; as such, pivaloyloxymethyl esters and similar esters are utilized in a conventional manner.

Owing to the close relationship between a free compound, a prodrug derivative and a salt compound, a certain compound in this invention also encompasses its prodrug derivative and salt form, where these are possible and appropriate.

The compounds of the formula (I) also include those compounds in which one or more atoms are replaced by their stable, non-radioactive isotopes; for example, a hydrogen atom by deuterium.

The compounds of formula (I) and (Ia), respectively, and their pharmaceutically useful salts reveal inhibitory activities on the enzymes beta-secretase, cathepsin D, plasmepsin II and/or HIV-protease.

The activitiy of inhibitors of beta-secretase, cathepsin D, plasmepsin II and/or HIV protease can be assessed experimentally with following *in vitro* assays.

The protease inhibitory activity of compounds can be tested with an assay kit using the fluorescence resonance energy transfer (FRET) technology and a recombinant i.e. baculovirus expressed enzyme preparation. The FRET is used to monitor the cleavage of the peptide substrate. The principle of the assay is as follows relies on a measurable energy difference, quantitatively depending on the presence of a peptide sequence. The peptide substrate is synthesized with two terminal fluorophores, a fluorescent donor and quenching acceptor. The distance between these two groups is selected so that upon light excitation, the donor fluorescence energy is significantly quenched by the acceptor through resonance energy transfer. Upon cleavage by the protease, the fluorophore is separated from the quenching group, restoring the fluorescence yield of the donor. Thus a weakly fluorescent peptide substrate becomes highly fluorescent upon enzymatic cleavage; the increase in fluorescence is linearly related to the rate of proteolysis.

The FRET assay was performed in white polysorp plates. The assay buffer consisted of 50 mM sodium acetate pH 5, 392 mM sodium chloride, 12.5% glycerol and 0.1 % BSA. The incubates per well were composed of 160 ul buffer, 10 ul inhibitor in DMSO, 10 ul peptide substrate in DMSO and 20 ul enzyme-solution. The inhibitors are tested in a concentration range of 1 pM to 1 mM. The fluorescently marked donor and acceptor peptide substrates are generated by solid phase peptide synthesis (Applied Biosystems). The beta-secretase peptide substrate Rh-Glu-Val-Asn-Leu-Asp-Ala-Glu-Phe-Lys-Quencher is obtained from Invitrogen, Carlsbad, CA, USA. The cathepsin D peptide substrate of the sequence DABCYL-Pro-Thr-Glu-Phe-Phe-Arg-Leu-OXL, the plasmepsin peptide substrate of the sequence DABCYL-Glu-Arg-Nle-Phe-Leu-Ser-Phe-Pro-OXL and the HIV protease peptide substrate of the sequence DABCYL-His-Lys-Ala-Arg-Val-Leu-Tyr-Glu-Ala-Nle-Ser-EDANS are all obtained from AnaSpec Inc, San Jose, CA, USA. The recombinantly expressed enzyme preparations are added in various amounts to the assay systems eg the beta-sectrase concentration is 1 unit/ml incubation volume, the cathepsin D concentration is 100 ng/ml, the HIV protease concentration is 500 ng/ml and the plasmepsin II concentration is 50 ng/ml. The reaction is started upon addition of the enzyme solution. The incubation occurs at 37°C over 30-120 min ie specifically the beta-secretase incubation lasts 60 min, the cathepsin D incubation 120 min, the plasmepsin II incubation 40 min and the HIV protease incubation 40 min. The reactions are stopped by the addition of 20 µl of a 1.0 M Tris Base solution. The enzymatic substrate to product conversion is assessed by fluorescence measurements at 460 nm wave length.

### In vitro enzyme inhibitory activities

The compounds of the present invention revealed structure-dependent and enzyme-specific inhibitory activities. The inhibitory activities were measured as IC50 values. Thus the beta-secretase inhibitory activity ranged between 1 pM and 1 mM; the values for cathepsin D ranged between 1 pM und 1 mM, for plasmepsin II between 1 pM und 1 mM and for HIV-protease between 1 pM und 1 mM.

The compounds of the formula (I) and the pharmaceutically usable salts thereof may find use as medicines, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formula (I) and pharmaceutically usable salts thereof may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.

Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

Subject of the present invention is also the use of the compounds of formula (I) and (Ia), respectively, and their pharmaceutically useful salts for the prevention, delay of progression or the treatment of Alzheimer Disease, malaria or HIV infection.

Subject of the present invention is also the use of the compounds of formula (I) and (la), respectively, and their pharmaceutically useful salts for the manufacture of a medication for the prevention, delay of progression or the treatment of Alzheimer Disease, malaria or HIV infection.

Subject of the present invention is also the method for the prevention, delay of progression or the treatment of Alzheimer Disease, malaria or HIV infection, whereby a therapeutically effective dose of a compound of the general formula (I) or preferred formula (Ia) is applied.

Subject of the present invention is also a pharmaceutical preparation that contains for the inhibition of beta-secretase, cathepsin D, plasmepsin and/or HIV-protease a compound of the general formula (I), or preferred of formula (la) as well as commonly used ingredients.

Subject of the present invention is also a pharmaceutical preparation for the prevention, delay of progression or treatment of Alzheimer Disease, malaria and HIV infection that contains a compound of the general formula (I), or preferred of formula (Ia) as well as commonly used ingredients.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

The examples which follow are intended to illustrate the present invention, but not to restrict it in any way. All temperatures are reported in degrees Celsius, pressures in mbar. Unless stated otherwise, the reactions take place at room temperature. The abbreviation "Rf = xx (A)" means, for example, that the Rf value xx is obtained in the solvent system A. The ratio of the solvents relative to one another is always reported in parts by volume. Chemical names of end products and intermediates were obtained with the aid of the program AutoNom 2000 (Automatic Nomenclature).

The following examples are prepared as described in detail in WO2005/070871, pages 20 to 43, which description is herewith incorporated.

### Examples:

1 N-(4(S)-Amino-5(S)-hydroxy-2(S)-isopropyl-6-piperidin-1-ylhexyl)-2-(3-methoxypropoxy)benzamide dihydrochloride
2 N-[4(S)-Amino-6-(2,2-dimethylpropionylamino)-5(S)-hydroxy-2(S)-isopropylhexyl]-2-(3-methoxypropoxy)benzamide hydrochloride
3 N-{4(S)-Amino-5(S)-hydroxy-2(S)-isopropyl-6-[2-methyl-2-(tetrahydropyran-4-yl)-propionylamino]hexyl}-2-(3-methoxypropoxy)benzamide hydrochloride
4 N-[4(S)-Amino-5(S)-hydroxy-2(S)-isopropyl-6-(2-oxopiperidin-1-yl)hexyl]-2-(3-methoxy-propoxy)-benzamide hydrochloride
5 N-[4(S)-Amino-5(S)-hydroxy-2(S)-isopropyl-6-(propane-2-sulphonylamino)hexyl]-2-(3-methoxypropoxy)benzamide hydrochloride
6 N-(4(S)-amino-5(S)-hydroxy-2(S)-isopropyl-6-piperidin-1-ylhexyl)-2-(4-methoxy-butoxy)benzamide dihydrochloride
7 N-[4(S)-amino-6-(2,2-dimethylpropionylamino)-5(S)-hydroxy-2(S)-isopropylhexyl]-2-(4-methoxybutoxy)benzamide hydrochloride
8 N-{4(S)-amino-5(S)-hydroxy-2(S)-isopropyl-6-[2-methyl-2-(tetrahydropyran-4-yl)-propionylamino]hexyl}-2-(4-methoxybutoxy)benzamide hydrochloride
9 N-[4(S)-amino-5(S)-hydroxy-2(S)-isopropyl-6-(2-oxopiperidin-1-yl)hexyl]-2-(4-methoxybutoxy)benzamide hydrochloride
10 N-[4(S)-amino-5(S)-hydroxy-2(S)-isopropyl-6-(propane-2-sulphonylamino)hexyl]-2-(4-methoxybutoxy)benzamide hydrochloride
11 N-(4(S)-amino-5(S)-hydroxy-2(S)-isopropyl-6-morpholin-4-ylhexyl)-2-(4-methoxy-butoxy)benzamide dihydrochloride
12 N-[4(S)-amino-6-(9-azabicyclo[3.3.1]non-9-yl)-5(S)-hydroxy-2(S)-isopropylhexyl]-2-(4-methoxybutoxy)benzamide dihydrochloride
13 N-[4(S)-amino-6-(cis-2,6-dimethylpiperidin-1-yl)-5(S)-hydroxy-2(S)-isopropylhexyl]-2-(4-methoxybutoxy)benzamide dihydrochloride
14 N-[4(S)-amino-6-(3-methylpiperidin-1-yl)-5(S)-hydroxy-2(S)-isopropylhexyl]-2-(4-methoxybutoxy)benzamide dihydrochloride
15 N-[4(S)-amino-6-(4-methylpiperidin-1-yl)-5(S)-hydroxy-2(S)-isopropylhexyl]-2-(4-methoxybutoxy)benzamide dihydrochloride
16 N-(4(S)-amino-6-sec-(S)-butylamino-5(S)-hydroxy-2(S)-isopropylhexyl)-2-(4-methoxy-butoxy)benzamide dihydrochloride
17 N-[4(S)-amino-6-(3,3-dimethyl-2-oxopyrrolidin-1-yl)-5(S)-hydroxy-2(S)-isopropylhexyl]-2-(4-methoxybutoxy)benzamide hydrochloride
18 N-(4(S)-amino-6-tert-butylamino-5(S)-hydroxy-(S)-isopropylhexyl)-2-(4-methoxy-butoxy)benzamide dihydrochloride
19 N-(4(S)-amino-5(S)-hydroxy-2(S)-isopropyl-6-isopropylaminohexyl)-2-(4-methoxy-butoxy)benzamide dihydrochloride
20 N-(4(S)-amino-6-sec-(R)-butylamino-5(S)-hydroxy-2(S)-isopropylhexyl)-2-(4-methoxy-butoxy)benzamide dihydrochloride
21 N-[4(S)-amino-6-(cyclopropylmethylamino)-5(S)-hydroxy-2(S)-isopropylhexyl]-2-(4-methoxybutoxy)benzamide dihydrochloride
22 N-[4(S)-amino-6-(1,1-dimethylpropylamino)-5(S)-hydroxy-2(S)-isopropylhexyl]-2-(4-methoxybutoxy)benzamide dihydrochloride
23 N-(4(S)-amino-6-ethylamino-5(S)-hydroxy-2(S)-isopropylhexyl)-2-(4-methoxybutoxy)-benzamide dihydrochloride
24 N-(4(S)-amino-5(S)-hydroxy-2(S)-isopropyl-6-propylaminohexyl)-2-(4-methoxy-butoxy)benzamide dihydrochloride
25 N-[4(S)-amino-6-(1-ethylpropylamino)-5(S)-hydroxy-2(S)-isopropylhexyl]-2-(4-methoxybutoxy)benzamide dihydrochloride
26 N-(4(S)-amino-6-cyclopentylamino-5(S)-hydroxy-2(S)-isopropylhexyl)-2-(4-methoxy-butoxy)benzamide dihydrochloride
27 N-[4(S)-amino-5(S)-hydroxy-2(S)-isopropyl-6-(2(R)-methylpiperidin-1-yl)hexyl]-2-(4-methoxybutoxy)benzamide dihydrochloride
28 N-[4(S)-amino-5(S)-hydroxy-2(S)-isopropyl-6-(2(S)-methylpiperidin-1-yl)hexyl]-2-(4-methoxybutoxy)benzamide dihydrochloride
29 N-(4(S)-amino-5(S)-hydroxy-6-isobutylamino-2(S)-isopropylhexyl)-2-(4-methoxy-butoxy)benzamide dihydrochloride
30 N-[4(S)-amino-6-(1-ethyl-1-methylpropylamino)-5(S)-hydroxy-2(S)-isopropylhexyl]-2-(4-methoxybutoxy)benzamide dihydrochloride
31 N-(4(S)-amino-6-cyclopropylamino-5(S)-hydroxy-2(S)-isopropylhexyl)-2-(4-methoxy-butoxy)benzamide dihydrochloride
32 N-(4(S)-amino-6-azepan-1-yl-5(S)-hydroxy-2(S)-isopropylhexyl)-2-(4-methoxybutoxy)-benzamide dihydrochloride
33 N-[4(S)-amino-5(S)-hydroxy-2(S)-isopropyl-6-(1(S)-methylpentylamino)hexyl]-2-(4-methoxybutoxy)benzamide dihydrochloride
34 N-[4(S)-amino-5(S)-hydroxy-2(S)-isopropyl-6-(1(R)-methylpentylamino)hexyl]-2-(4-methoxybutoxy)benzamide dihydrochloride
35 N-(4(S)-amino-5(S)-hydroxy-2(S)-isopropyl-6-pyrrolidin-1-ylhexyl)-2-(4-methoxy-butoxy)benzamide dihydrochloride
36 N-(4(S)-amino-6-benzylamino-5(S)-hydroxy-2(S)-isopropylhexyl)-2-(4-methoxy-butoxy)benzamide dihydrochloride
37 N-[4(S)-amino-5(S)-hydroxy-2(S)-isopropyl-6-(2-oxoazepan-1-yl)hexyll-2-(4-methoxy-butoxy)benzamide hydrochloride
38 N-[4(S)-amino-6-(3,3-dimethyl-2-oxoazepan-1-yl)-5(S)-hydroxy-2(S)-isopropylhexyl]-2-(4-methoxybutoxy)benzamide hydrochloride
39 N-[4(S)-amino-6-(2-cyclohexyl-2-methylpropionylamino)-5(S)-hydroxy-2(S)-isopropyl-hexyl]-2-(4-methoxybutoxy)benzamide hydrochloride
40 N-{4(S)-amino-5(S)-hydroxy-2(S)-isopropyl-6-[(1-phenylcyclobutanecaronyl)amino]-hexyl}-2-(4-methoxybutoxy)benzamide hydrochloride
41 N-[4(S)-amino-6-(2,2-dimethylhexanoylamino)-5(S)-hydroxy-2(S)-isopropylhexyl]-2-(4-methoxybutoxy)benzamide hydrochloride
42 N-(4(S)-amino-6-{[1-(4-chlorophenyl)cyclobutanecarbonyl]amino}-5(S)-hydroxy-2(S)-isopropylhexyl)-2-(4-methoxybutoxy)benzamide hydrochloride
43 N-[4(S)-amino-5(S)-hydroxy-2(S)-isopropyl-6-(2-methyl-2-m-tolylpropionylamino)-hexyl]-2-(4-methoxybutoxy)benzamide hydrochloride
44 N-[4(S)-amino-6-(2-cyclopentyl-2-methylpropionylamino)-5(S)-hydroxy-2(S)-isopropyl-hexyl]-2-(4-methoxybutoxy)benzamide hydrochloride
45 N-[4(S)-amino-5(S)-hydroxy-2(S)-isopropyl-6-(2-methyl-2-morpholin-4-ylpropionyl-amino)hexyl]-2-(4-methoxybutoxy)benzamide dihydrochloride
46 N-{4(S)-amino-6-[2-(3-fluorophenyl)-2-methylpropionylamino]-5(S)-hydroxy-2(S)-isopropylhexyl}-2-(4-methoxybutoxy)benzamide hydrochloride
47 N-{4(S)-amino-6-[(1-cyclohexylcyclobutanecarbonyl)aminol-5(S)-hydroxy-2(S)-isopropylhexyl}-2-(4-methoxybutoxy)benzamide hydrochloride
48 N-[4(S)-amino-5(S)-hydroxy-2(S)-isopropyl-6-(2-methyl-2-pyridin-3-ylpropionylamino)-hexyl]-2-(4-methoxybutoxy)benzamide dihydrochloride
49 N-[4(S)-amino-6-(3-chloro-2,2-dimethylpropionylamino)-5(S)-hydroxy-2(S)-isopropylhexyl]-2-(4-methoxybutoxy)benzamide hydrochloride
50 N-[6-(2-acetylamino-2-methylpropionylamino)-4(S)-amino-5(S)-hydroxy-2(S)-isopropylhexyl]-2-(4-methoxybutoxy)benzamide hydrochloride
51 N-{4(S)-amino-5(S)-hydroxy-2(S)-isopropyl-6-[(1-trifluoromethylcyclobutanecarbonyl)-amino]hexyl}-2-(4-methoxybutoxy)benzamide hydrochloride
52 N-[4(S)-amino-6-(2-cyclohexyloxy-2-methylpropionylamino)-5(S)-hydroxy-2(S)-isopropylhexyl]-2-(4-methoxybutoxy)benzamide hydrochloride
53 N-[4(S)-amino-5(S)-hydroxy-2(S)-isopropyl-6-(2-methoxy-2-methylpropionylamino)-hexyl]-2-(4-methoxybutoxy)benzamide hydrochloride
54 N-[4(S)-amino-5(S)-hydroxy-2(S)-isopropyl-6-(2-methyl-2-piperidin-1-ylpropionylamino)hexyll-2-(4-methoxybutoxy)benzamide dihydrochloride
55 N-{4-amino-5-hydroxy-2-isopropyl-6-[(1-methylcyclohexanecarbonyl)amino]hexyl}-2-(4-methoxvbutoxv)benzamide hydrochloride
56 N-{4(S)-amino-5(S)-hydroxy-6-[2-(1H-indol-3-yl)-2-methylpropionylamino]-2(S)-isopropylhexyl}-2-(4-methoxybutoxy)benzamide hydrochloride
57 N-[4(S)-amino-5(S)-hydroxy-2(S)-isopropyl-6-(2-methoxypropionylamino)hexyl]-2-(4-methoxvbutoxv)benzamide hydrochloride
58 N-(3(S)-amino-2(S)-hydroxy-5(S)-{[2-(4-methoxybutoxy)benzoylamino]methyl}-6-methylheptyl)adamantane-1-carboxamide hydrochloride
59 N-{4(S)-amino-6-[(2,2-dimethylpropionyl)hydroxyamino]-5(S)-hydroxy-2(S)-isopropylhexyl}-2-(4-methoxybutoxy)benzamide hydrochloride
60 N-(4(S)-amino-6-cyclopropanesulphonylamino-5(S)-hydroxy-2(S)-isopropylhexyl)-2-(4-methoxvbutoxv)benzamide hydrochloride
61 N-[4(S)-amino-5(S)-hydroxy-6-(4-hydroxy-2-oxopyrrolidin-1-yl)-2(S)-isopropylhexyl]-2-(4-methoxvbutoxv)benzamide hydrochloride
62 N-[4(S)-amino-5(S)-hydroxy-2(S)-isopropyl-6-(2-oxotetrahydropyrimidin-1-yl)hexyl]-2-(4-methoxybutoxy)benzamide hydrochloride
63 N-[4(S)-amino-6-(3,3-dimethylureido)-5(S)-hydroxy-2(S)-isopropylhexyl]-2-(4-methoxvbutoxv)benzamide hydrochloride
64 N-(4(S)-amino-6-benzoylamino-5(S)-hydroxy-2(S)-isopropylhexyl)-2-(4-methoxybutoxy)benzamide hydrochloride
65 N-(4(S)-amino-5(S)-hydroxy-2(S)-isopropyl-6-phenylmethanesulphonylaminohexyl)-2-(4-methoxybutoxy)benzamide hydrochloride
66 N-[4(S)-amino-5(S)-hydroxy-2(S)-isopropyl-6-(2(R)-methoxymethylpyrrolidin-1-yl)-hexyl]-2-(4-methoxybutoxy)benzamide hydrochloride
67 N-[4(S)-amino-6-(formylisopropylamino)-5(S)-hydroxy-2(S)-isopropylhexyl]-2-(4-methoxybutoxy)benzamide hydrochloride
68 N-[4(S)-amino-5(S)-hydroxy-2(S)-isopropyl-6-(thiophene-2-sulphonylamino)hexyl]-2-(4-methoxybutoxy)benzamide hydrochloride
69 N-(4(S)-amino-6-benzenesulphonylamino-5(S)-hydroxy-2(S)-isopropylhexyl)-2-(4-methoxybutoxy)benzamide hydrochloride
70 N-[6-(acetylmethylamino)-4(S)-amino-5(S)-hydroxy-2(S)-isopropylhexyl]-2-(4-methoxybutoxy)benzamide hydrochloride
71 N-[4(S)-amino-6-(1-carbamoylethylamino)-5(S)-hydroxy-2(S)-isopropylhexyl]-2-(4-methoxybutoxy)benzamide dihydrochloride
72 N-[6-(3(S)-acetylaminopyrrolidin-1-yl)-4(S)-amino-5(S)-hydroxy-2(S)-isopropylhexyl]-2-(4-methoxybutoxy)benzamide hydrochloride
73 N-[4(S)-amino-5(S)-hydroxy-2(S)-isopropyl-6-(2-methyl-2-pyridin-2-ylpropionylamino)-hexyl]-2-(4-methoxybutoxy)benzamide dihydrochloride
74 N-[4(S)-amino-5(S)-hydroxy-2(S)-isopropyl-6-(2-methyl-2-piperidin-4-ylpropionylamino)hexyl]-2-(4-methoxybutoxy)benzamide dihydrochloride
75 N-[4(S)-amino-5(S)-hydroxy-2(S)-isopropyl-6-(3,3,3-trifluoro-2(R)-methoxy-2-phenylpropionylamino)hexyl]-2-(4-methoxybutoxy)benzamide hydrochloride
76 N-[6-(N-acetylhydrazino)-4(S)-amino-5(S)-hydroxy-2(S)-isopropylhexyl]-2-(4-methoxybutoxy)benzamide dihydrochloride
77 N-[4(S)-amino-5(S)-hydroxy-2(S)-isopropyl-6-(2(R)-methoxy-3-phenylpropionylamino)hexyl]-2-(4-methoxybutoxy)benzamide hydrochloride
78 N-[4(S)-amino-6-(3-cyclohexyl-2(R)-methoxypropionylamino)-5(S)-hydroxy-2(S)-isopropylhexyl]-2-(4-methoxybutoxy)benzamide hydrochloride
79 N-[4(S)-amino-5(S)-hydroxy-2(S)-isopropyl-6-(2-methyl-2-piperidin-3(R)-ylpropionylamino)hexyl]-2-(4-methoxybutoxy)benzamide dihydrochloride
80 N-[4(S)-amino-5(S)-hydroxy-2(S)-isopropyl-6-(2-methyl-2-piperidin-3(S)-ylpropionylamino)hexyl]-2-(4-methoxybutoxy)benzamide dihydrochloride
81 N-{4(S)-amino-5(S)-hydroxy-6-[2-(1H-imidazol-4-yl)-2-methylpropionylamino]-2(S)-isopropylhexyl}-2-(4-methoxybutoxy)benzamide hydrochloride
82 N-[4(S)-amino-6-(2,2-dimethyl-4-methylaminobutyrylamino)-5(S)-hydroxy-2(S)-isopropylhexyl]-2-(4-methoxybutoxy)benzamide dihydrochloride
83 N-{4(S)-amino-5(S)-hydroxy-6-[(2(S)-hydroxy-(S)-cyclopentanecarbonyl)amino]-2(S)-isopropylhexyl}-2-(4-methoxybutoxy)benzamide hydrochloride
84 N-[4(S)-amino-5(S)-hydroxy-2(S)-isopropyl-6-(2-methyl-2-piperidin-2(S)-ylpropionylamino)hexyl]-2-(4-methoxybutoxy)benzamide dihydrochloride
85 N-[4(S)-amino-5(S)-hydroxy-2(S)-isopropyl-6-(2-methyl-2-piperidin-2(R)-ylpropionylamino)hexyl]-2-(4-methoxybutoxy)benzamide dihydrochloride
86 N-[4(S)-amino-5(S)-hydroxy-2(S)-isopropyl-6-(2-methyl-2-(1,2-dihydrospiro[3H-indole-3,4'-piperidin]-1'-yl)propionylamino)hexyl]-2-(4-methoxybutoxy)benzamide dihydrochloride
87 N-{4(S)-amino-5(S)-hydroxy-6-[2-(cis-4-hydroxycyclohex-1-yl)-2-methylpropionylamino]-2(S)-isopropylhexyl}-2-(4-methoxybutoxy)benzamide hydrochloride
   The starting materials are prepared as follows:
   a) 2-(cis-4-Hydroxy-cyclohexyl)-2-methyl-propionic acid
      0.200 g of 2-(cis-4-hydroxy-cyclohexyl)-2-methyl-propionic acid methyl ester are dissolved in 4 ml of methanol. 4 ml of a 1M aqueous lithium hydroxide solution are added and the mixture is stirred for 16 hours at room temperature. The reaction mixture is then neutralised with 1 M HCl and concentrated by evaporation. The title compound is identified from the residue by means of flash chromatography (SiO2 60F) based on its Rf value.
   b) 2-(cis-4-Hydroxy-cyclohexyl)-2-methyl-propionic acid methyl ester and 2-(trans-4-Hydroxy-cyclohexyl)-2-methyl-propionic acid methyl ester
      A solution of 2.0 g of 2-(cis/trans-4-hydroxy-cyclohexyl)-2-methyl-propionic acid in 40 ml of methanol is cooled to 0°C. 20 ml of a 2M trimethysilyldiazomethane solution in hexanes are added dropwise and the reaction solution is left to stand at room temperature for 1 hour. The solution is concentrated under reduced pressure and the residue taken up in ethyl acetate. The solution is washed with saturated aqueous sodium carbonate solution and brine, dried over sodium sulphate and concentrated by evaporation. The residue is purified by flash chromatography (SiO2 60F) to provide the title compounds as colourless oils, the cis isomer eluting first. Rf (cis) = 0.11 (1:3 EtOAc-heptane); Rf (trans) = 0.09 (1:3 EtOAc-heptane).
   c) 2-(cis/trans-4-Hydroxy-cyclohexyl)-2-methyl-propionic acid
      2.690 g of 2-(4-hydroxy-phenyl)-2-methyl-propionic acid (29913-51-7) are dissolved in 20 ml of water and 30 ml of 1 M NaOH solution. 0.200 g of Raney-Nickel are added and the reaction mixture is hydrogenated at 50 bar and 150°C for 24 hours. The catalyst is removed by filtration over Hyflo and the filtrate is concentrated by evaporation. The residue is taken up in 200 ml of water and the solution neutralized with 1 M HCI to pH 6. The reaction mixture is then extracted with dichloromethane (2x200 ml) and ethyl acetate (2x20 ml) and the combined organic phases are dried over sodium sulphate and concentrated by evaporation to provide the title compounds as a ca. 1:4 mixture of cis/trans-isomers. The white solid is used for the next step without further purification.
88 N-{4(S)-amino-5(S)-hydroxy-6-[2-(trans-4-hydroxycyclohex-1-yl)-2-methylpropionylamino]-2(S)-isopropylhexyl}-2-(4-methoxybutoxy)benzamide hydrochloride
89 N-{4(S)-amino-5(S)-hydroxy-2(S)-isopropyl-6-[2-(cis-4-methoxycyclohex-1-yl)-2-methylpropionylamino]hexyl}-2-(4-methoxybutoxy)benzamide hydrochloride
   The starting materials are prepared as follows:
   a) 2-(cis-4-Methoxy-cyclohexyl)-2-methyl-propionic acid
      0.200 g of 2-(cis-4-methoxy-cyclohexyl)-2-methyl-propionic acid methyl ester are dissolved in 4 ml of methanol. 4 ml of a 1 M aqueous lithium hydroxide solution is added and the mixture is stirred for 16 hours at room temperature. The reaction mixture is then neutralised with 1 M HCl and concentrated under reduced pressure The title compound is identified from the residue by means of flash chromatography (SiO2 60F) based on its Rf value.
   b) 2-(cis-4-Methoxy-cyclohexyl)-2-methyl-propionic acid methyl ester
      0.500 g of 2-(cis-4-hydroxy-cyclohexyl)-2-methyl-propionic acid methyl ester (Example 87b) are dissolved in 5 ml of dry tetrahydrofuran. 0.120 g of sodium hydride (60% dispersion) is added in portions and the mixture stirred at 40°C for 1 hour. Methyl iodide (0.233 ml) is added and the mixture heated to 40°C for 5 hours. The reaction mixture is then cooled to room temperature, quenched with 5 ml of water and extracted with tert-butyl methyl ether (2x50 ml). The combined organic phases are dried over sodium sulphate and concentrated by evaporation. The title compound is identified from the residue by means of flash chromatography (SiO2 60F) based on its Rf value.
90 N-{4(S)-amino-5(S)-hydroxy-2(S)-isopropyl-6-[2-(trans-4-methoxycyclohex-1-yl)-2-methylpropionylamino]hexyl}-2-(4-methoxybutoxy)benzamide hydrochloride
91 N-[4(S)-amino-6-(2-cyclohexyl-2(R)-methoxyacetylamino)-5(S)-hydroxy-2(S)-isopropylhexyl]-2-(4-methoxybutoxy)benzamide hydrochloride
   The starting material is prepared as follows:
   a) (R)-Cyclohexyl-methoxy-acetic acid
      An autoclave is charged with a solution of 1.00 g of (R)-α-methoxy-phenyl acetic acid in 20 ml methanol. 0.100 g of Nishimura catalyst are added and the mixture is hydrogenated at 4 bar and 20°C for 1 hour. The mixture is filtered over Hyflo and the filtrate concentrated by evaporation to provide the title compound as a colourless oil. The crude material is used without further purification. Rf = 0.84 (150:54:10:1 dichloromethane-methanol-water-acetic acid).
92 N-[4(S)-amino-5(S)-hydroxy-2(S)-isopropyl-6-(2(R)-methoxy-2-phenylacetylamino)-hexyl]-2-(4-methoxybutoxy)benzamide hydrochloride
93 N-[4(S)-amino-5(S)-hydroxy-2(S)-isopropyl-6-(2(R)-methoxy-3,3-dimethylbutyrylamino)hexyl]-2-(4-methoxybutoxy)benzamide hydrochloride
94 N-[4(S)-amino-5(S)-hydroxy-2(S)-isopropyl-6-(3,3,3-trifluoro-2-methoxy-2-trifluoromethylpropionylamino)hexyl]-2-(4-methoxybutoxy)benzamide hydrochloride
95 N-[4(S)-amino-5(S)-hydroxy-2(S)-isopropyl-6-(3,3,3-trifluoro-2(R)-methoxy-2-methylpropionylamino)hexyl]-2-(4-methoxybutoxy)benzamide hydrochloride
96 N-[4(S)-amino-5(S)-hydroxy-2(S)-isopropyl-6-(3,3,3-trifluoro-2(S)-methoxy-2-methylpropionylamino)hexyl]-2-(4-methoxybutoxy)benzamide hydrochloride
97 N-[4(S)-amino-6-(2-cyciohexyl-3,3,3-trifiuoro-2(R)-methoxypropionylamino)-5(S)-hydroxy-2(S)-isopropylhexyl]-2-(4-methoxybutoxy)benzamide hydrochloride
98 N-[4(S)-amino-5(S)-hydroxy-2(S)-isopropyl-6-(2(R)-methoxy-2-phenylpropionylamino)hexyl]-2-(4-methoxybutoxy)benzamide hydrochloride
99 N-[4(S)-amino-6-(2-cyclohexyl-2(R)-methoxypropionylamino)-5(S)-hydroxy-2(S)-isopropylhexyl]-2-(4-methoxybutoxy)benzamide hydrochloride
100 N-{4(S)-amino-5(S)-hydroxy-2(S)-isopropyl-6-[(1-methoxycyclopentanecarbonyl)-amino]hexyl}-2-(4-methoxybutoxy)benzamide hydrochloride
101 N-{4(S)-amino-5(S)-hydroxy-2(S)-isopropyl-6-[(1-methoxycyclohexanecarbonyl)-amino]hexyl}-2-(4-methoxybutoxy)benzamide hydrochloride
102 N-[4(S)-Amino-5(S)-hydroxy-2(S)-isopropyl-6-(2-methyl-2-piperidin-3(R,S)-ylpropionylamino)-hexyl]-2-(4-methoxy-butoxy)-benzamide dihydrochloride
103 N-{4(S)-Amino-5(S)-hydroxy-2(S)-isopropyl-6-[2-methyl-2-(1-methyl-piperidin-3(R,S)-yl)-propionylamino]-hexyl}-2-(4-methoxy-butoxy)-benzamide dihydrochloride
104 N-[4(S)-Amino-5(S)-hydroxy-2(S)-isopropyl-6-(2-methyl-2-piperidin-2(R,S)-ylpropionylamino)-hexyl]-2-(4-methoxy-butoxy)-benzamide dihydrochloride
105 N-{4(S)-Amino-5(S)-hydroxy-2(S)-isopropyl-6-[2-methyl-2-(1-methyl-piperidin-2(R,S)-yl)-propionylamino]-hexyl}-2-(4-methoxy-butoxy)-benzamide dihydrochloride
106 N-{4(S)-Amino-5(S)-hydroxy-6-[2(R,S)-(trans-2-hydroxy-cyclohexyl)-2-methylpropionylamino]-2(S)-isopropyl-hexyl}-2-(4-methoxy-butoxy)-benzamide hydrochloride
107 N-{4(S)-Amino-5(S)-hydroxy-6-[2-(3(S)-hydroxy-cyclohex-1(R)-yl)-2-methylpropionylamino]-2(S)-isopropyl-hexyl}-2-(4-methoxy-butoxy)-benzamide hydrochloride
108 N-[4(S)-Amino-5(S)-hydroxy-6-(2-imidazol-1-yl-2-methyl-propionylamino)-2(S)-isopropyl-hexyl]-2-(4-methoxy-butoxy)-benzamide dihydrochloride
109 N-[4(S)-Amino-6-(2-cyano-2,2-dimethyl-acetylamino)-5(S)-hydroxy-2(S)-isopropylhexyl]-2-(4-methoxy-butoxy)-benzamide hydrochloride
110 N-{6-[trans-2-(4-Acetylamino-cyclohexyl)-2-methyl-propionylaminol-4(S)-amino-5(S)-hydroxy-2(S)-isopropyl-hexyl}-2-(4-methoxy-butoxy)-benzamide hydrochloride
111 N-{6-[2-(3(S)-Acetylamino-cyclohex-1(R)-yl)-2-methyl-propionylamino]-4(S)-amino-5(S)-hydroxy-2(S)-isopropyl-hexyl}-2-(4-methoxy-butoxy)-benzamide hydrochloride
112 N-[4(S)-Amino-6-(2,2-difluoro-2-phenyl-acetylamino)-5(S)-hydroxy-2(S)-isopropylhexyl]-2-(4-methoxy-butoxy)-benzamide hydrochloride
113 N-[4(S)-Amino-6-(2-cyclohexyl-2,2-difluoro-acetylamino)-5(S)-hydroxy-2(S)-isopropylhexyl]-2-(4-methoxy-butoxy)-benzamide hydrochloride
114 N-{4(S)-Amino-6-[2,2-difluoro-2-(tetrahydro-pyran-4-yl)-acetylamino]-5(S)-hydroxy-2(S)-isopropyl-hexyl}-2-(4-methoxy-butoxy)-benzamide hydrochloride
115 N-[4(S)-Amino-5(S)-hydroxy-2(S)-isopropyl-6-(2-methyl-propane-2-sulfonylamino)-hexyl]-2-(4-methoxy-butoxy)-benzamide hydrochloride
116 N-[4(S)-Amino-6-(2-cyclohexyl-propane-2-sulfonylamino)-5(S)-hydroxy-2(S)-isopropyl-hexyl]-2-(4-methoxy-butoxy)-benzamide hydrochloride

## Claims

1. Use of a compound of formula 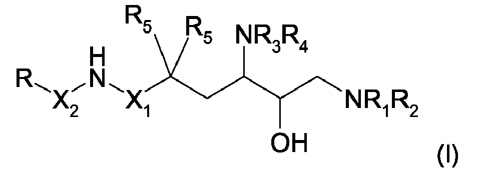 R₁ is a) hydrogen, hydroxyl or amino; or
is b) C₁-C₈-alkyl, C₃-C₈-cycloalkyl, C₁-C₈-alkanoyl, C₁-C₈-alkoxycarbonyl, aryl-C₀-C₄-alkyl or heterocyclyl-C₀-C₄-alkyl, which radicals may be substituted by 1-4 C₁-C₈-alkyl, halogen, cyano, oxide, oxo, trifluoromethyl, C₁-C₈-alkoxy, C₁-C₈-alkoxycarbonyl, aryl or heterocyclyl;
R₂ is a) C₁-C₈-alkyl, C₃-C₈-cycloalkyl, C₁-C₈-alkylsulphonyl, C₃-C₈-cycloalkylsulphonyl, aryl-C₀-C₈-alkylsulphonyl, heterocyclylsulphonyl, C₃-C₁₂-cycloalkyl-C₁-C₈-alkanoyl, C₃-C₁₂-cycloalkyl-C₃-C₈-cycloalkanoyl, aryl-C₁-C₈-alkanoyl, aryl-C₃-C₈-cycloalkanoyl, C₁-C₈-alkanoyl, C₁-C₈-alkoxycarbonyl, optionally N-mono- or N,N-di-C₁-C₈-alkylated carbamoyl-C₀-C₈-alkyl, aryl-C₀-C₄-alkyl or heterocyclyl-C₀-C₄-alkyl, which radicals may be substituted by 1-4 C₁-C₈-alkyl, C₃-C₈-cycloalkyl, C₃-C₈-cycloalkoxy, amino, C₁₋₆-alkylamino, di-C₁₋₆-alkylamino, C₀-C₆-alkylcarbonylamino, halogen, cyano, hydroxyl, oxide, oxo, trifluoromethyl, C₁-C₈-alkoxy, optionally N-mono- or N,N-di-C₁-C₈-alkylated carbamoyl, C₁-C₈-alkoxycarbonyl, C₁₋₆-alkylenedioxy, aryl or heterocyclyl; or
is b) together with R₁ and the nitrogen atom to which they are bonded, a saturated or partly unsaturated 4-8-membered heterocyclic ring which may contain an additional nitrogen, oxygen or sulphur atom or an -SO- or -SO2- group, in which case the additional nitrogen atom may optionally be substituted by C₁-C₈-alkyl, C₁-C₈-alkanoyl, C₁-C₈-alkoxycarbonyl, aryl or heterocyclyl radicals, and this heterocyclic ring may be part of a bicyclic or tricyclic ring system having a total of up to 16 members, and the second ring may also contain a nitrogen, oxygen or sulphur atom or an -SO- or -SO2- group, and the nitrogen atom of the second ring may optionally be substituted by C₁-C₈-alkyl, C₁-C₈-alkanoyl, C₁-C₈-alkoxycarbonyl, aryl or heterocyclyl radicals and all ring systems mentioned may be substituted by 1-4 C₁-C₈-alkyl, halogen, hydroxyl, oxide, oxo, trifluoromethyl, C₁-C₈-alkoxy, C₁-C₈-alkoxy-C₁-C₈-alkyl, C₁-C₈-alkoxy-C₁-C₈-alkoxy, C₁-C₈-alkoxycarbonylamino, C₁-C₈-alkylcarbonylamino, C₁-C₈-alkylamino, N,N-di-C₁-C₈-alkylamino, aryl-C₀-C₄-alkyl, aryloxy-C₀-C₄-alkyl, aryl-C₀-C₄-alkyl-C₁-C₈-alkoxy, aryloxy-C₀-C₄-alkyl-C₁-C₈-alkoxy, heterocyclyl-C₀-C₄-alkyl, heterocyclyloxy-C₀-C₄-alkyl, heterocyclyl-C₀-C₄-alkyl-C₁-C₈-alkoxy or heterocyclyloxy-C₀-C₄-alkyl-C₁-C₈-alkoxy;
R₃ is hydrogen, C₁-C₈-alkyl, C₁-C₈-alkoxycarbonyl or C₁-C₈-alkanoyl;
R₄ is hydrogen, C₁-C₈-alkyl, C₁-C₈-alkoxycarbonyl or C₁-C₈-alkanoyl;R₅ are each independently hydrogen or C₁-C₈-alkyl, or, together with the carbon atom to which they are bonded, are a C₃-C₈-cycloalkylidene radical;
R is an optionally substituted unsaturated carbocyclic or heterocyclic radical; one of the X₁ and X₂ radicals is carbonyl and the other is methylene; or pharmaceutically usable salt thereof,
for the preparation of a medication for the inhibition of beta-secretase, cathepsin D, plasmepsin II and/or HIV-protease

2. Use according to claim 1 and specified as formula (Ia) 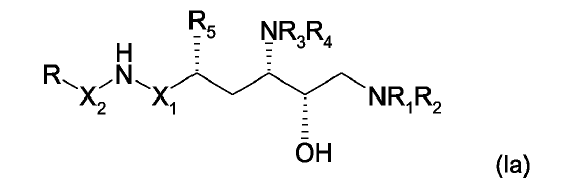 where R, R₁, R₂, R₃, R₄, R₅, X₁ and X₂ are each as defined in Claim 1.

3. Use of a compound of the general formula (I) and (Ia), according to the claims 1 and 2 for the preparation of a medication for the prevention, delay of progression or treatment of Alzheimer Disease, malaria or HIV infection.
